# EUROPÄISCHE PATENTANMELDUNG

(11) **EP 4 205 562 A2**
(43) Veröffentlichungstag der Anmeldung: **05.07.2023**
(21) Anmeldenummer: 22217051.6
(22) Anmeldetag: 28.12.2022
(51) Int. Cl.: A23L 33/185, A23K 20/147, A61Q 11/00

(54) **ZAHNPFLEGEFUTTERMITTELZUSAMMENSETZUNG**

(30) Priorität: 28.12.2021 LU 501109
(71) Anmelder: Pferdesport Fuchs GbR, 38165 Lehre Niedersachsen (DE)
(72) Erfinder: Fuchs, Thomas, 38165 Lehre (DE); Fuchs, Kathrin, 38165 Lehre (DE)
(74) Vertreter: Gruner, Leopold Joachim

(57) **Zusammenfassung**

Die vorliegende Erfindung betrifft eine Zahnpflegefuttermittelzusammensetzung zur Förderung des Wohlbefindens eines Tieres sowie insbesondere zu seiner Zahnpflege, wobei die Zahnpflegefuttermittelzusammensetzung zumindest eine erste, zweite und dritte Komponente umfasst, wobei die erste Komponente einem Abrieb von Zahnmaterial förderlich ist, die zweite Komponente einen Futtermittelbestandteil umfasst und die dritte Komponente ein verzehrbares Haftmittel umfasst, das dazu eingerichtet ist, die erste und die zweite Komponente miteinander zu verbinden und wobei das Haftmittel aus einem getreidebasierten Haftmittel mit einem Zuckeranteil von unter 2 % ausgewählt ist.

## Beschreibung

### TECHNISCHES GEBIET

Die vorliegende Erfindung betrifft eine Zahnpflegefuttermittelzusammensetzung zur Förderung des Wohlbefindens eines Tieres sowie insbesondere zu seiner Zahnpflege.

### STAND DER TECHNIK

Es ist bekannt, dass Haustiere (z.B. Hunde, Katzen, Meerschweinchen, Nagetiere) sowie Nutztiere (z.B. Pferde, Schweine, Rinder, Schafe, Ziegen) einen Spieltrieb haben. Diesen zu unterbinden, indem dem Tier keine Möglichkeit gegeben wird dem Spieltrieb nachzukommen, kann sich negativ auf das psychische Wohlbefinden des Tieres auswirken und Verhaltensstörungen bewirken.

Gleichzeitig ist es notwendig, dass das Tier in der Lage ist Zahnpflege zu betreiben, um bspw. der Bildung von Plaques und Bakterienbefall vorzubeugen. Dies wird beispielsweise mit Tierfutter gelöst, dass aufgrund seiner rauen Beschaffenheit die Oberfläche der Zähne reinigen soll.

Jedoch wird dem Tierfutter auch Industriezucker beigemengt, der als günstiges Füllmaterial dient. Zudem soll durch die Beimengung von karamellisiertem Zucker eine ansprechende bräunliche Färbung und ein angenehmerer Geruch des Tierfutters erreicht werden. Nachteilig ist jedoch, dass das Tier somit einer vermehrten Zuckeraufnahme ausgesetzt ist, was sich negativ auf die Gesundheit des Tieres auswirkt. So ist bekannt, dass auch Tiere durch eine erhöhte Zuckerzufuhr zu Diabetes neigen und zudem das Zahnmaterial geschädigt wird.

Insbesondere die Zähne von Equiden (z.B. Pony, Pferd, Esel, Maultier, Muli, Zebra) werden heutzutage nicht mehr so beansprucht, wie es biologisch vorgesehen ist. Pferde, die ursprünglich Steppenbewohner waren, ernährten sich ausschließlich von silikatreichem Steppengras, welches für ein Abnutzen der Zähne gesorgt hat, so dass innerhalb eines Jahres bis etwa 3 mm Zahnmaterial von den Schneidezähnen und etwa 5 bis 8 mm Zahnmaterial von den Backenzähnen abgerieben wurde.

Um die Länge des abgeriebenen Zahnmaterials schieben sich die Zähne aus dem Zahnfach in die Maulhöhle wieder nach. Jedoch verbringen domestizierte Pferde heute durchschnittlich weit weniger Zeit mit der Aufnahme von Futter, wodurch folglich weniger Zahnmaterial abgerieben wird. Zudem ist das verwendete Futter oft nicht hart genug, um die Zähne genügend abzunutzen.

Bei Equiden kommen zwei Arten der Zahnerkrankungen vor, die primär auf die Nahrungsaufnahme zurückzuführen sind, die Infundibular-Karies und die periphere Karies. Dabei sind vor allem vergärende Nahrungsmittelreste, welche lange im Maul verbleiben sowie zuckerhaltige Nahrungsmittel, wie aus Melasse oder Melassierten nahrungsmitteln, die von Mikroorganismen zu Säuren abgebaut werden. Diese demineralisiert die Zähne und lässt sie brüchig werden.

Um Zahnprobleme zu vermeiden, muss daher in regelmäßigen Abständen, meist einmal jährlich, ein Tierarzt die Maulhöhle begutachten und den Abrieb simulieren. Hierfür sind eine Sedierung und entsprechendes zahntechnisches Equipment (Maulgatter, Raspel, Schleifmaschine) notwendig. Die Zähne werden durch Abschleifen gekürzt und Unregelmäßigkeiten und scharfe Kanten glattgeraspelt.

Verzichten Pferdebesitzer auf diese Behandlung, bilden sich scharfe Kanten, Wellen und Haken an den Pferdezähnen. So kann das Pferd sein Futter nicht mehr ausreichend zerkleinern. Zudem verletzen die scharfen Kanten die Schleimhäute, was zu Entzündungen und Infektionen führen kann.

Um einem Pferd eine Möglichkeit zu bieten, den Spieltrieb zu befriedigen und gleichzeitig Zahnmaterial abzutragen, werden diesem sogenannte Knabberhölzer (bspw. aus Birkenholz) zur Verfügung gestellt. Bei diesen handelt es sich in der Regel um in kurze Abschnitte gesägte Baumstämme mit geringem Durchmesser von bis etwa 20 cm, oder um Äste, Zweige und dergleichen.

Es ist auch bekannt, dass die Knabberhölzer mit einer klebenden Schicht versehen werden, um bspw. Blüten oder Sämereien an diesen zu befestigen, um diese attraktiver für das Pferd zu machen. Beispielhaft sei hier das Produkt "Hotzenklotz" der gleichnamigen Firma genannt.

Jedoch ist die klebende Schicht aus einer stark zuckerhaltigen Substanz gebildet und ist insoweit nachteilig für die Zahngesundheit des daran knabbernden Tieres. Weiterhin können die verwendeten Hölzer Pilze und Schimmel aufweisen. Mit Schimmelpilzen kontaminiertes Futter führt bei Pferden zu schweren Leber- und Nierenerkrankungen, kann Koliken auslösen sowie die Fruchtbarkeit schädigen. Besonders nachteilig ist dabei die Verwendung von Haftmitteln, die kurzkettige Kohlenhydrate, insbesondere Zucker enthalten, da diese den gegenteiligen Effekt durch Förderung von Mikroorganismen, die Karies durch Säureausscheidung verursachen, die Zahngesundheit der Equiden beeinträchtigen.

### AUFGABE

Die Erfindung hat es sich zur Aufgabe gemacht, eine Futtermittelzusammensetzung bereitzustellen, die das Tierwohl, insbesondere die Zahngesundheit eines Tieres fördert, ohne, dass das Tier durch den Konsum der Futtermittelzusammensetzung eine vermehrte Zuckeraufnahme oder eine Aufnahme von schädlichen Pilzen, insbesondere Schimmel, erwirkt.

### LÖSUNG

Die Aufgabe wird mit einer Zahnpflegefuttermittelzusammensetzung zur Förderung des Wohlbefindens eines Haustieres und/oder Nutztieres, insbesondere zu dessen Zahnpflege mit den Merkmalen des Anspruchs 1 erfüllt. Weitere vorteilhafte Ausgestaltungen sind den Unteransprüchen, der Beschreibung und den Ausführungsbeispielen zu entnehmen.

Vorzugsweise wird eine Zahnpflegefuttermittelzusammensetzung zur Förderung des Wohlbefindens eines Tieres, insbesondere zur Zahnpflege und Nahrungsergänzung eines Tieres, bereitgestellt, die zumindest folgende drei Komponenten umfasst:
- eine erste Komponente, insbesondere einen Grundkörper, die/der zumindest ein Material umfasst oder daraus besteht, welches einem Abrieb von Zahnmaterial förderlich ist, insbesondere zum Abrieb des Zahnmaterials des Tieres geeignet ist, und wobei das Material vorzugsweise als einteiliger Formkörper ausgeführt ist,
- eine zweite Komponente, insbesondere ein Futtermitteladditiv, die/das zumindest einen Futtermittelbestandteil umfasst,
- eine dritte Komponente, insbesondere ein Haftmittel, die/das ein verzehrbares Haftmittel umfasst, oder daraus besteht, welches zur stoffschlüssigen Verbindung aller Komponenten eingerichtet ist,
wobei das Haftmittel ein getreidebasiertes Haftmittel, insbesondere ein Haftmittel auf Biopolymerbasis ist, wobei das Haftmittel vorzugsweise aus einer Pflanze extrahiert wird, und wobei das Haftmittel einen Zuckergehalt von weniger als 2 % aufweist.

### ALLGEMEINE VORTEILE

Das zuckerfreie, verzehrbare und biologische Haftmittel erlaubt eine stoffschlüssige Verbindung der beteiligten Komponenten und kann von einem Tier aufgenommen werden, ohne dass mit der Aufnahme zuckerbedingte Krankheitsbilder, insbesondere eine Verschlechterung des Zahnbildes, einhergeht. Darüber hinaus fungiert der Klebstoff allein und insbesondere in Kombination mit der Zusammensetzung als Nahrungsergänzungsmittel, mit dem gesundheitliche Aspekte positiv beeinflusst werden können.

Weitere Vorteile sind der Beschreibung und den Ausführungsbeispielen zu entnehmen.

### BESCHREIBUNG DER ERFINDUNG

Die Erfindung betrifft eine Zahnpflegefuttermittelzusammensetzung zur Förderung des Wohlbefindens eines Tieres, insbesondere zu dessen Zahnpflege.

Unter einem Wohlbefinden eines Tieres wird hierin verstanden, dass es seinem Spieltrieb nachkommen kann, was sich vorteilhaft auf dessen Verhalten auswirkt, um somit Langweile zu verhindern, da gelangweilte Tiere zu Verhaltensstörungen tendieren. Weiterhin ist von einem Wohlbefinden auch die Nahrungsaufnahme eines Tieres umfasst, das dieses ohne Nahrung ebenso gesundheitlich beeinträchtigt ist.

Bei einem Haustier und/oder Nutztier handelt es sich um jede Form eines domestizierten Tieres, vorzugsweise um ein Säugetier (bspw. Hunde, Katzen, Meerschweinchen, Nagetiere, Pferde, Schweine, Rinder, Schafe, Ziegen). Weiterhin ist von einem Haus- oder Nutztier auch ein Tier in Gefangenschaft (z.B. ein in einem Zoo, Tierpark, Auffangstation, Gnadenhof, Pferdeklappe untergebrachtes Tier) umfasst. Im Sinne der Erfindung soll ein Mensch ebenso als ein Tier verstanden werden.

Unter einer Zahnpflege wird hierin ein Vorgang verstanden, welcher dafür sorgt, dass durch den bestimmungsgemäßen Gebrauch (Aufnahme) der Zahnpflegefuttermittelzusammensetzung das Zahnmaterial in einer für das Tier günstigen Art und Weise abgetragen wird. Im Wesentlichen hat das Tier Einfluss darauf, wie es die Zahnpflegefuttermittelzusammensetzung konsumiert.

Die Zahnpflegefuttermittelzusammensetzung umfasst zumindest drei Komponenten. Eine erste Komponente, insbesondere ein Grundköper, umfasst ein Material, oder besteht daraus, welches einem Abrieb von Zahnmaterial förderlich ist, wobei der Fachmann entscheiden kann, welches Material für den bestimmungsgemäßen Gebrauch zu verwenden ist. So hängt dies bspw. von der Spezies des Tieres ab, für welche die erfindungsgemäße Zahnpflegefuttermittelzusammensetzung verwendet werden soll.

Vorzugsweise dient die erste Komponente, insbesondere der Grundkörper, zugleich als formgebende Komponente, insbesondere als einteiliges formgebendes Element, der Zahnpflegefuttermittelzusammensetzung. Vorzugsweise ist die erste Komponente, insbesondere der Grundkörper, als Formkörper ausgebildet. Der Begriff "Formkörper" bezeichnet im Sinne dieser Erfindung einen Gegenstand von konstruktiver Gestalt, dem diese aktiv gegeben wurde, und der z.B. durch spanlose Formung (z.B. durch Pressen, Pressspritzen oder Spritzgießen) in allseitig geschlossenen Werkzeugen hergestellt worden ist. Alternativ dazu ist der Formkörper ein gewollt geformter Körper/Gegenstand in Form eines Schafts, bspw. als Element einer verholzten Hauptachse einer Baumpflanze. Insbesondere ist innerhalb der Zahnpflegefuttermittelzusammensetzung die erste Komponente, insbesondere der Grundkörper, als einteiliger Formkörper ausgebildet, d.h., die erste Komponente, insbesondere der Grundkörper, ist nicht als partikulärer und/oder kleinteiliger Gegenstand ausgebildet bzw. in der Zahnpflegefuttermittelzusammensetzung verteilt. Dies hat den Vorteil, dass hierdurch einerseits dem Tier gegenüber eine möglichst naturgetreue Umgebung nachgebildet ist und darüber hinaus die erste Komponente, insbesondere der Grundkörper, in partikulärer Form nicht ungewollt aus der Zahnpflegefuttermittelzusammensetzung freigelegt werden kann und dadurch unerwünschter Weise freigesetzt bzw. verteilt wird (bspw. auf den Boden fällt) und dadurch dem Tier nicht mehr zur Zahnpflege und/oder zur Nahrungsergänzung zur Verfügung steht. In einer bevorzugten Ausführungsform ist das Material der ersten Komponente, insbesondere des Grundkörpers als einteiliger Formkörper ausgebildet.

In einer bevorzugten Ausführungsform liegt in der Zahnpflegefuttermittelzusammensetzung die erste Komponente, insbesondere der Grundkörper, gegenüber dem Haftmittel im Gewichtsüberschuss vor, insbesondere im Gewichtsverhältnis von zumindest 3:1, besonders bevorzugt im Gewichtsverhältnis von zumindest 5:1, ganz besonders bevorzugt im Gewichtsverhältnis von zumindest 10:1 gegenüber dem Haftmittel.

Die erste Komponente, insbesondere der Grundkörper, umfasst vorzugweise ein Kernmaterial oder besteht aus diesem Kernmaterial. Vorzugsweise ist auf der Oberfläche der ersten Komponente, insbesondere des Grundkörpers, zumindest die dritte Komponente, insbesondere das Haftmittel angeordnet, wobei die dritte Komponente vorzugsweise zumindest teilweise oder vollständig als Schicht, besonders bevorzugt als durchgängige Schicht auf zumindest einer Oberfläche der ersten Komponente (bspw. einer Seitenfläche, einer Stirnseite und/oder Mantelfläche des Grundkörpers) angeordnet ist.

Einem Veterinär oder Dentist im tiergesundheitlichen Bereich ist bekannt, was für ein Grad an Abrieb von Zahnmaterial für die jeweilige Spezies vorteilhaft ist.

Vorzugsweise ist die erste Komponente, insbesondere der Grundköper, ein Schaft, besonders bevorzugt als ein Element einer verholzten Hauptachse einer Baumpflanze ausgebildet oder besteht daraus. Dies hat den Vorteil, dass für die Formgebung der ersten Komponente keine aufwendigen formgebenden Herstellungsschritte vorgenommen werden müssen. Vielmehr kann ein solcher Schaft, insbesondere ein Element einer verholzten Hauptachse einer Baumpflanze, einfach durch Zerteilen eines Stamms, bspw. eines Stamms aus Holz, gewonnen werden. In einer Ausführungsform ist das Material der ersten Komponente, insbesondere des Grundköpers, aus einem Holz wie sibirischem Birkenholz gebildet oder besteht daraus. Sibirisches Birkenholz ist schwerer und härter als deutsches Birkenholz, womit bevorzugt ein erhöhter Abrieb am Zahnmaterial von bspw. Pferden erreicht wird. Weitere mögliche Ausführungsformen umfassen als Material für die erste Komponente, insbesondere in Form als formgebenden Grundkörper ein Erlenholz, Hainbuchenholz, Haselnussholz, Pappelholz, Ulmenholz.

Es ist auch denkbar, dass die erste Komponente, insbesondere ein Grundköper, ein Gestein und/oder ein Mineral (z.B. Kalisalz) und/oder eine mineralische Verbindung umfasst, um vorteilhaft einen Abrieb von Zahnmaterial zu ermöglichen.

Weiterhin umfasst die Zahnpflegefuttermittelzusammensetzung eine zweite Komponente, insbesondere ein Futtermitteladditiv, welche zumindest einen Futtermittelbestandteil umfasst. Ein Futtermittelbestandteil umfasst oder besteht aus Stoffen, Stoffverbindungen, Materialien, oder Organismen, von welchen sich heterotrophe Spezies ernähren. Dabei richtet sich die stoffliche Zusammensetzung eines Futtermittelbestandteiles an die Bedürfnisse der Spezies, welche diesen aufnehmen soll, wobei diese dem Fachmann bekannt sind, der diese Zusammensetzung auswählt.

Ein Futtermittelbestandteil kann bspw. Blüten, Kräuter, Sämereien umfassen. Weiterhin kann ein erfindungsgemäßer Futtermittelbestandteil auch zumindest ein Inhaltsstoff sein, wie er sich in gängigen Futtermittelzusammensetzungen oder -mischungen wiederfindet. Beispielhaft seien hier genannt: Fleisch, Tiermehl, Getreide, Obst, Früchte.

Weiterhin umfasst die Zahnpflegefuttermittelzusammensetzung als eine dritte Komponente ein verzehrbares Haftmittel. Unter einem Haftmittel wird hierin ein Stoff, eine Stoffverbindung, oder Materialzusammensetzung verstanden, die dazu eingerichtet ist, eine stoffschlüssige Verbindung zumindest einer der vorgenannten Komponenten zu ermöglichen.

In einer bevorzugten Ausführungsform umfasst die Zahnpflegefuttermittelzusammensetzung zur Förderung des Wohlbefindens eines Tieres, insbesondere zu dessen Zahnpflege, zumindest folgende drei Komponenten: Eine erste Komponente, insbesondere ein Grundkörper, die zumindest ein Material umfasst oder daraus besteht, welches zum Abrieb des Zahnmaterials geeignet ist, einem Abrieb von Zahnmaterial förderlich ist, eine zweite Komponente, insbesondere ein Futtermitteladditiv, die zumindest einen Futtermittelbestandteil umfasst oder daraus besteht sowie mindestens eine dritte Komponente, insbesondere ein Haftmittel, die ein verzehrbares Haftmittel umfasst oder daraus besteht, welches zur stoffschlüssigen Verbindung aller Komponenten eingerichtet ist, wobei das Haftmittel ein Biopolymer-basiertes Haftmittel ist.

Bevorzugt ist ein Aufbau für die Zahnpflegefuttermittelzusammensetzung, bei dem zumindest eine Oberfläche der ersten Komponente, insbesondere des formgebenden Grundkörpers (bspw. eine Seitenfläche, eine Stirnseite und/oder Mantelfläche davon) mit der dritten Komponente, insbesondere dem Haftmittel, teilweise oder vollständig umgeben ist und außen (d.h. auf der Außenseite der dritten Komponente, insbesondere des Haftmittels, insbesondere der als Kontaktfläche zur ersten Komponente dienenden Seite abgewandten Seite) das Futtermitteladditiv adhäsiert ist. Dies hat insbesondere den Vorteil, dass zur Herstellung der Zahnpflegefuttermittelzusammensetzung die dritte Komponente, insbesondere das Haftmittel einfach auf die erste Komponente, insbesondere den formgebenden Grundkörper, aufgetragen (bspw. durch Bestreichen) aufgebracht werden kann und anschließend (d.h. auch noch im Nachhinein) das jeweils vom Nutzer gewünschte bzw. erforderliche Futtermitteladditiv auf die dritte Komponente, insbesondere das Haftmittel, aufgebracht werden kann. In diesem Fall handelt es sich bei dem Aufbau der Zahnpflegefuttermittelzusammensetzung um einen Quasi-Schicht-Aufbau, bei dem im Zentrum die erste Komponente, insbesondere der formgebende Grundkörper, angeordnet ist, auf den die dritte Komponente, insbesondere das Haftmittel, angeordnet ist, auf welcher wiederum das Futtermitteladditiv angeordnet ist.

In einer bevorzugten Ausführungsform der vorliegenden Erfindung wird ein physikalisch abbindendes Haftmittel verwendet. Physikalisch abbindende Klebstoffe bestehen aus einer Lösung oder Dispersion von Polymeren, die durch Verdampfen des Lösungsmittels, bevorzugt Wasser, ihre Haftwirkung aufbauen.

In einer besonders bevorzugten Ausführungsform werden erfindungsgemäß Polymere verwendet, die in der Natur vorkommen oder von lebenden Organismen hergestellt werden, sogenannte Biopolymere. Bevorzugt handelt es sich um Polymere von Sacchariden, besonders bevorzugt um Polymere von Glucose, insbesondere Polymere die mit einer β-1,4-glycosidischen und/oder α-1,4-glycosidischen und/oder einer α-1,6-glycosidischen Bindung vernetzt sind. Dies hat den Vorteil, dass eine Verzehrfähigkeit gewährleistet ist und die Ausgangsstoffe für den Haftstoff sehr preiswert erhältlich sind. In einem Aspekt kann durch die Verwendung von zertifiziertem pflanzlichem Ausgangsmaterial aus ökologischem Anbau (z. B. EU-Bio-Label) die erfindungsgemäße Zahnpasta auch als biologisch nachhaltiges Produkt verkauft werden, was eine höhere wirtschaftliche Attraktivität mit sich bringt.

In einer besonders bevorzugten Ausführungsform handelt es sich bei dem Biopolymer um eine Mischung aus α-1,4-glycosidischen und/oder einer α-1,6-glycosidischen Polymeren der Glucose, besonders bevorzugt um eine Stärke, die aus einem pflanzlichen Produkt isoliert wurde, insbesondere bevorzugt ein Stärkeextrakt aus einem Getreide, ganz besonders bevorzugt eine Hafer-Stärke. Diese Biopolymere sind nicht nur verträglich, sondern auch kostengünstig erhältlich.

In einem Aspekt der Erfindung handelt es sich bei dem Biopolymer um eine Mischung aus α-1,4-glycosidischen und/oder einer α-1,6-glycosidischen Polymeren der Glucose sowie weiteren Inhaltstoffen, die mit der Stärke extrahiert werden, zum Beispiel Proteine, Vitamine und β-Glucan, sogenannte Stärke-Extrakte. Besonders bevorzugt sind dabei Extrakte, die aus einem pflanzlichen Produkt isoliert wurde, insbesondere bevorzugt ein Stärkeextrakt aus einem Getreide, ganz besonders bevorzugt eine Hafer-Stärke. Diese Extrakte sind nicht nur verträglich, sondern auch kostengünstig erhältlich und haben gegenüber reiner isolierter Stärke Vorteile, wie einen höheren Nährwert, die Verfügungstellung wichtiger Spurenelemente und essenzieller Aminosäuren.

In einer alternativen Ausführungsform können modifizierte Biopolymere, wie Methylierte Stärken oder Cellolosefasern wie Methylcellulose eingesetzt werden. Diese Fasern sind im Vergleich zu den nicht modifizierten varianten schwerer verdaulich und setzen weniger Kohlenhydrate frei, was zu einer besseren Verdauung oder der Regulierung des Blutzuckers eines Tieres genutzt werden kann.

In einer Ausführungsform ist das Haftmittel getreidebasiert. In einer Ausführungsform ist das getreidebasierte Haftmittel ein Biopolymer basiertes Haftmittel.

Bevorzugt kann dieses Haftmittel von einem Tier aufgenommen und von diesem zumindest teilweise verdaut werden oder das Haftmittel wird unverdaut wieder ausgeschieden. In einer besonders bevorzugten Ausführungsform ist das Haftmittel für die Verdauung förderlich, da es einen hohen Anteil an schwer verdaulichen Biopolymeren enthält. In einer Ausführungsform senkt das Haftmittel den Cholesterinspiegel eines Tieres, insbesondere durch die enthaltene β-Glucane, die ebenfalls einen Beitrag zur Haftwirkung haben. Haftwirkung und gesundheitliche Wirkung gehen also Hand in Hand, ganz im Gegensatz zum bisherigen Stand der Technik, der erstarrende Zucker als Klebstoffe verwendet.

In einer besonders bevorzugten Ausführungsform handelt es sich bei dem verzehrbaren Haftmittel um ein getreidebasiertes Haftmittel. Ein solches wird aus einem Getreide (bspw. Weizen, Gerste, Hafer), ganz bevorzugt aus Hafer, wie weiter unten beschrieben, gewonnen. Dies hat den Vorteil, dass Getreide zur klassischen Diät von Equiden dient und so eine höhere Verträglichkeit gewährleistet.

Hafer enthält sehr feinkörnige Stärke, wodurch das Herauslösen der Stärke in einer hydrophilen Flüssigkeit, insbesondere Wasser, bereits aus Haferkörnern und/oder Haferflocken gut möglich ist. Haferstärke besitzt eine höhere Schwellfähigkeit und Löslichkeit als Mehlstärke, was vorteilhaft für die Herstellung der flüssigen Haftmasse ist, da weniger Wasser benötigt wird, was erfindungsgemäß zu einer besseren Trocknung führt.

In einer bevorzugten Ausführungsform liegt die Partikelgröße, insbesondere der mittlere Partikeldurchmesser (d₅₀), der Stärke-Partikel des pflanzlichen Ausgangsmaterials bei 1-15 µm, besonders bevorzugt bei 1-12 µm. Die Stärke-Partikel sind besonders bevorzugt polyedrisch (vielflächig), oval bis unregelmäßig geformt, insbesondere polyedrisch, bis unregelmäßig geformt, aber nicht rund, was die Löslichkeit verbessert, da sich aus der polyedrischen bis unregelmäßigen Form/Geometrie der Stärke-Partikel eine größere spezifische Oberfläche ergibt, was die haftvermittelnden Eigenschaften der dritten Komponente, insbesondere des Haftmittels, verbessert. Stärke-Partikel mit den erfindungsgemäßen Formen/Geometrien und Partikelgrößen finden sich bevorzugt in Mais, Reis, Erbsen, Hirse, Amarant und/oder Hafer, insbesondere in Hirse, Amarant und/oder Hafer. Beispielsweise sind die Stärke-Partikel in Hafer auf einer Seite rund und auf der gegenüberliegenden Seite polyedrisch ausgebildet. Insbesondere hat die hierin beschriebene Kombination aus der Partikelgröße und der Formgebung der Stärke-Partikel eine hohe spezifische Oberfläche der Stärke-Partikel, insbesondere der aus einem Extrakt einer Pflanze gewonnen Stärke-Partikel, von mehr als 0,6 m²/g, insbesondere von mehr als 0,8 m²/g, besonders bevorzugt von mehr als 1,0 m²/g, ganz besonders bevorzugt von mehr als 1,1 m²/g, zur Folge, wobei zur Ermittlung der spezifischen Oberfläche der Stärke-Partikel zumindest ein physikalisches Modell basierend auf BET-Adsorptionsisothermen verwendet wird, wodurch vorteilhaft die haftvermittelnden Eigenschaften der dritten Komponente, insbesondere des Haftmittels, erhöht ist. Zum Vergleich sind Stärke-Partikel im Weizen rund ausgebildet, wobei die Partikelgröße im Bereich von 20-30 µm liegt, sodass diese Stärke-Partikel lediglich eine spezifische Oberfläche unterhalb von 0,6 m²/g aufweisen.

Die Stärke-Partikel in Hafer weisen eine durchschnittliche/mittlere Partikelgröße (d₅₀) von 2-11 µm, deutlich kleiner als bei anderen Getreidesorten wie Weizen (20-30 µm), auf, wodurch die Herstellung einer wässrigen Lösung und/oder Dispersion deutlich erleichtert ist. In Reis und in Mais beträgt die durchschnittliche Partikelgröße der Stärke-Partikel insbesondere 3-8 µm bzw. 2-15 µm. Erbsen weisen darüber hinaus ebenfalls geringe durchschnittliche Partikelgrößen mit einer eng verteilten Partikelgrößenverteilung im Bereich von 5-10 µm auf. Aufgrund einer geringen Partikelgröße der Stärke-Partikel können vorteilhaft mehr Stärke-Partikel in demselben Volumen einer dritten Komponente angeordnet sein, was die haftvermittelnden Eigenschaften pro Volumeneinheit der dritten Komponente, insbesondere des Haftmittels, erhöht. In einer bevorzugten Ausführungsform ist die dritte Komponente, insbesondere der Haftvermittler daher ausgewählt aus einem Hafer-basierten, Reis-basierten, Mais-basierten, Amarant-basiertes, Hirse-basierten und/oder Erbsen-basierten Haftmittel. Besonders bevorzugt weist die dritte Komponente ein Hafer-basiertes, Amarant-basiertes und/oder Hirse-basiertes Haftmittel auf oder besteht daraus.

In einer bevorzugten Ausführungsform weist das Biopolymer, insbesondere die Stärke, besonders bevorzugt eine aus Hafer, Amarant und/oder Hirse gewonnene Stärke einen Lipidgehalt (Gew.-%) von vorzugsweise mindestens 1,5 Gew.-%, am meisten bevorzugt von mindestens 2 Gew.-% auf.

Die nachfolgende Tabelle gibt eine Übersicht zu Eigenschaften von Stärke-Partikeln typischer natürlicher Quellen.

**Tabelle 1: Physikalisch chemische Eigenschaften verschiedener Stärke-Partikel**

| **Quelle** | **Partikelgröße** | **Spezifische Oberfläche** | **Lipidanteil (Gew.-%)** | **Geometrie** |
|---|---|---|---|---|
| Hafer | 2-11 µm | 1,224 m²/g | 2,09-2,45 | polyedrisch, oval bis unregelmäßig |
| Reis | 3-8 µm | 1,267 m²/g | 1,21 | polyedrisch |
| Erbsen | 5-10 µm | 0,698 m²/g | 0,64 | nierenförmig |
| Mais | 2-15 µm | 0,687 m²/g | 0,97 | rund, polyedrisch |
| Hirse | 1-10 µm | 1,65 m²/g | 2,60-2,90 | polyedrisch, raue Kanten |
| Amarant | 1-4 µm | 2,75 m²/g | 1,50 | polyedrisch |
| Weizen | 20-30 µm | 0,534 m²/g | 0,88 | rund, linsenförmig |
| Gerste | 20-25 µm | 0,599 m²/g | 0,70 | linsenförmig |
| Roggen | 10-40 µm | 0,383 m²/g | 0,62 | linsenförmig |

**Tabelle 2: Gelatinierungeigenschaften verschiedener Stärke-Partikel**

| **Quelle** | **Anfangstemperatur T₀ (°C)** | **Peaktemperatur Tₚ (°C)** | **Endtemperatur T_{c} (°C)** | **Gel Enthalpy ΔH (J/g)** |
|---|---|---|---|---|
| Hafer^{[a]} | 60.0 | 66.0 | 71.0 | 10.03-12.83 |
| Hafer^{[b]} | 91.0-92.4 | 99.0-102.2 | --- | 0.27-0.72 |
| Reis | 62.94 | 69.82 | 78.69 | 8.70 |
| Erbsen | 53.61 | 58.79 | 62.78 | 3.75 |
| Kartoffel | 61.1 | 65.0 | 65.0 | 3.5 |
| Mais | 66.63 | 71.74 | 78.84 | 9.53 |
| Hirse | 71.25 | 78.26 | 82.44 | 8.48 |
| Amarant | 69,3 | 74,9 | 83,2 | 10,6 |
| Weizen | 57.6 | 58.1 | 58.1 | 12.1 |

| | | | | |
|---|---|---|---|---|
| [a]: Niedertemperatur-Endotherme. [b]: Hochtemperatur-Endotherme. | | | | |

Darüber hinaus zeichnet sich ein Hafer-basiertes, Reis-basiertes, Mais-basiertes, Hirse-basiertes, Amarant-basiertes und/oder Erbsen-basiertes Haftmittel, insbesondere ein Hafer-basiertes, Reis-basiertes, Hirse-basiertes, Amarant-basiertes und/oder Erbsen-basiertes Haftmittel, ganz besonders bevorzugt ein Hafer-basiertes, Amarant-basiertes und/oder Hirse-basiertes Haftmittel, dadurch aus, dass sich darin die transformierte Stärke nicht oder nur sehr langsam zurückbildet, wodurch die haftvermittelnden Eigenschaften lange anhalten. Vorteilhaft ist ein höhere Lipidgehalt von durchschnittlich etwa 1,0 Gew.-% (±0,05) und mehr, vorzugsweis von mehr als 1,0 Gew.-%, besonders bevorzugt von mehr als 1,5 Gew.-%, ganz besonders bevorzugt von mehr als 2,0 Gew.-% der dritten Komponente, insbesondere des Haftvermittlers, insbesondere der Stärke-Partikel in der dritten Komponente, ein wesentlicher Grund für die langsamere Retrogradation/Rückbildung der Stärke.

In einer Ausführungsform ist die Löslichkeit der Stärke in Wasser bei 95 °C über 30 %, besonders bevorzugt über 33 %, insbesondere bevorzugt über 35 %.

In einer Ausführungsform weist das Biopolymer, insbesondere die Stärke-Partikel, nach dem Aufschlämmen in Wasser und ggf. einem Wärmeeintrag eine Verkleisterungsenthalpie (-ΔH_{gel}) von zumindest 10 J pro g Stärke, insbesondere von zumindest 12 J pro g Stärke auf.

In einer besonders bevorzugten Ausführungsform liegt der Niedrigtemperatur Gelatinierungsbereich des verwendeten Biopolymers, bevorzugt einer Stärke, insbesondere der Stärke-Partikel, zwischen 50 und 80 °C, besonders bevorzugt zwischen 55 und 75 °C, am meisten bevorzugt zwischen 60 und 71 °C. Das Überschreiten dieses Gelatinierungsbereiches erlaubt eine Homogenisierung und Lösung des Biopolymers, wodurch es erfindungsgemäß auf dem Grundkörper aufgetragen eine ausreichende Haftwirkung aufbringen kann.

In einer alternativen Ausführungsform, in der das Biopolymer, insbesondere eine Stärke, einen Hochtemperatur Gelatinierungsbereich besitzt, liegt dieser bevorzugt zwischen 90 und 110°C, besonders bevorzugt zwischen 95 und 105 °C. Das Überschreiten dieses zweiten Gelatinierungsbereiches erlaubt eine noch bessere Homogenisierung und Lösung des Biopolymers im Extraktionslösungsmittel, wodurch es erfindungsgemäß auf dem Grundkörper aufgetragen eine ausreichende Haftwirkung aufbringen kann. Weiterhin kann es in diesem Lösungszustand besser filtriert und von nicht gelösten Bestandteilen abgetrennt werden.

Zudem hat sich insbesondere gezeigt, dass sich die Verkleisterungseigenschaften von Stärke-Partikeln vor allem in Abhängigkeit von der Partikelgröße ändern, wobei große Stärke-Partikel bei höheren Temperaturen zuerst gelatiniert werden, gefolgt von kleineren Stärke-Partikeln bei Verringerung der Temperaturen und bei Vorliegen eines breiteren Gelatinierungsbereichs, wodurch verbesserte Verkleisterungseigenschaften resultieren. Daher sind Stärke-Partikel und/oder Mischungen davon, vorzugsweise aus einem pflanzlichen Ausgangsmaterial, ganz besonders bevorzugt aus dem Extrakt einer Pflanze für die Bereitstellung bzw. Herstellung einer dritten Komponente, insbesondere eines Haftmittels, wie eines Biopolymer-basierten Haftmittels, wünschenswert, wobei die Stärke-Partikel und/oder Mischungen davon einen breiten Gelatinierungsbereich (ΔT = Tₚ - T₀) aufweisen, wobei der Gelatinierungsbereichs breiter ist als 10 K, wie bspw. bei Stärke-Partikeln enthalten in oder gewonnen aus Hafer, Reis, Mais, Amarant und/oder Hirse.

Aus den vorgenannten Gründen in Bezug auf die verbesserten Verkleisterungseigenschaften sind Stärke-Partikel und/oder Mischungen davon, vorzugsweise aus einem pflanzlichen Ausgangsmaterial, ganz besonders bevorzugt aus dem Extrakt einer Pflanze für die Bereitstellung bzw. Herstellung einer dritten Komponente, insbesondere eines Haftmittels, wie eines Biopolymer-basierten Haftmittels, bevorzugt, bei denen zumindest eine Peaktemperatur (Tₚ) oberhalb von 60 °C, besonders bevorzugt oberhalb von 65 °C, ganz besonders bevorzugt oberhalb von 70 °C , insbesondere oberhalb von 74 °C liegt, wie bspw. bei Stärke-Partikeln enthalten in oder gewonnen aus Hafer, Amarant und/oder Hirse.

Vorteilhaft muss für das Herauslösen der Stärke der Hafer, insbesondere die Haferkörner und/oder Haferflocken, Amarant und/oder die Hirse nicht zerkleinert werden, was die Abtrennung des Hafers, Amarant oder der Hirse nach dem Aufkochen von dem Sud erleichtert. Im Gegensatz zu anderen Stärkequellen, wie zum Beispiel Maisstärke muss in einem aufwendigen Verfahren gewonnen werden, in dem die Maiskörner für einige Tage in Kochsalzlösung quellen müssen, bevor er zerkleinert werden kann und die Stärke von den restlichen Bestandteilen abgetrennt werden kann. Dies hat den technischen Vorteil, dass das Haftmittel mit geringerem Aufwand gewonnen werden kann.

Ein weiterer Aspekt der Erfindung, bei dem Haferstärke als Haftvermittler eingesetzt wird, ist, dass Haferstärke im Gegensatz zu den meisten anderen Getreidesorten aufgrund der starken Protein-Stärke-Matrix und des Vorhandenseins von β-Glucan nicht ohne großen Aufwand als Reinsubstanz erhältlich ist. Die Nebenstoffe, Protein und β-Glucan können so dem Tier zur Verfügung gestellt werden, während bei anderen Stärkesorten abgetrennt und verworfen werden müssen.

Es hat sich gezeigt, dass eine Verringerung des Volumens der hydrophilen Flüssigkeit während des Schrittes des Herauslösens der Stärke aus dem Getreide zu einer verbesserten Klebewirkung des getreidebasierten Haftmittels führt. Dies kann auf eine Anreicherung der Konzentration der Stärke zurückgeführt werden.

Besonders bevorzugt erfolgt das Herauslösen der Stärke aus dem pflanzlichen Ausgangsmaterial, insbesondere aus dem Extrakt einer Pflanze, insbesondere aus einem Getreide, bei Temperaturen oberhalb von 70 °C, besonders bevorzugt oberhalb von 80 °C, ganz besonders bevorzugt im Bereich von 90 °C bis 110 °C, am meisten bevorzugt zwischen 95 °C bis 105 °C wobei die hydrophile Flüssigkeit, insbesondere Wasser zum Sieden erhitzt wird.

In einer bevorzugten Ausführungsform der Erfindung ist es eine herausragende Leistung der Erfinder, die Extraktion des Biopolymers oberhalb von 70 °C, besonders bevorzugt oberhalb von 80 °C, ganz besonders bevorzugt im Bereich von 90 °C bis 110 °C, am meisten bevorzugt zwischen 95 °C bis 105 °C durchzuführen, da in diesen Temperaturbereichen Biopolymere, insbesondere Stärke-Partikel (insbesondere mit einer Zusammensetzung bzw. einem Aufbau und/oder Eigenschaften wie hierin definiert) für die Verkleisterung oder Gelatinierung gewonnen bzw. extrahiert werden, besonders bevorzugt aus pflanzlichem Ausgangsmaterial, ganz besonders bevorzugt aus dem Extrakt einer Pflanze, insbesondere aus einem Getreide, gewonnen werden, die der dritten Komponente, insbesondere dem Haftmittel, ausgeprägte und langlebige haftvermittelnde Eigenschaften vermitteln. So liegt beispielsweise bei Haferstärke, Reisstärke, Maisstärke, Amarantstärke und Hirsestärke in diesen Bereichen die Peaktemperatur bzw. die Endtemperatur für den Gelierungsprozess der gewünschten Stärke-Partikel. Insbesondere tritt für Haferstärke in diesem Bereich bei hohen Temperaturen eine zweite Endotherme (Verkleisterung oder Gelatinierung) auf, bei der ein weiterer Gelierungsprozess einsetzt. Bei den hohen Temperaturen, insbesondere für Haferstärke, handelt es sich um das Aufbrechen intramolekularer Wechselwirkungen zwischen den Stärke-Polymeren. Durch die höhere Temperatur als bei anderen Kleistern erforderlich ist, wird eine bessere Fließeigenschaft und eine höhere Löslichkeit erreicht, wodurch der auf diese Art generierte Kleister besser verstreich fähig ist und eine bessere Haftung aufbauen kann, da es zu einer geringeren Aggregation und Verklumpung, insbesondere während der Herstellung der Zahnpflegefuttermittelzusammensetzung kommt.

Zudem hat sich insbesondere gezeigt, dass sich die Verkleisterungseigenschaften von Stärke-Partikeln vor allem in Abhängigkeit von der Partikelgröße ändern, wobei große Stärke-Partikel bei höheren Temperaturen zuerst gelatiniert werden, gefolgt von kleineren Stärke-Partikeln bei Verringerung der Temperaturen und bei Vorliegen eines breiteren Gelatinierungsbereichs, wodurch verbesserte Verkleisterungseigenschaften resultieren. Daher sind Stärke-Quellen, vorzugsweise ein pflanzliches Ausgangsmaterial, ganz besonders bevorzugt ein Extrakt einer Pflanze, insbesondere aus einem Getreide, für die Bereitstellung bzw. Herstellung einer dritten Komponente, insbesondere eines Haftmittels, wünschenswert, die Stärke mit einem breiten Gelatinierungsbereichs (ΔT = Tₚ - T₀) aufweisen, wobei der Gelatinierungsbereichs breiter ist, als 10 K, wie bspw. für Stärke-Partikeln enthalten in oder gewonnen aus Hafer, Reis, Mais, Amarant oder Hirse.

Nach einer bevorzugten Ausgestaltung der Erfindung erfolgt das Herauslösen der Stärke aus dem Getreide bei kontinuierlicher Durchmischung, bspw. mittels Rühren, Schütteln oder durch Eintrag von Energie mittels Ultraschall (bspw. durch eine Sonotrode). Dies sorgt für eine gute Durchmischung aller Komponenten, bspw. des Maises, des Reises, der Erbsen, der Hirse, des Amarants und/oder des Hafers, bevorzugt der Hirse, des Amarants und/oder des Hafers, insbesondere der Haferkörner und/oder Haferflocken in der hydrophilen Flüssigkeit und für eine verbesserte Freisetzung der Stärke in die hydrophile Flüssigkeit.

Nach dem Herauslösen der Stärke aus dem Getreide werden die verbleibenden Feststoffpartikel des Getreides von der Flüssigkeit abgetrennt. Dies kann beispielsweise mit einem Sieb oder einem feinporigen Netz, bspw. mit einer Nutsche oder einem feinmaschigen Gewebe (bspw. eine Mullwindel) erfolgen.

Haferkörner enthalten für gewöhnlich zwischen 55 Gew.-% und 62,5 Gew.-% Kohlenhydrate, ca. 13 Gew.-% Eiweiß, 10 Gew.-% Ballaststoffe, 7 Gew.-% Fett sowie ca. 3 Gew.-% Mineralstoffe und Vitamine. Zudem enthält Hafer mit 500 mg pro 100 g einen, verglichen mit anderen Getreidearten, hohen Lysin-Gehalt. Lysin zählt zu den limitierenden Aminosäuren und ist essentiell für den Leber- und Muskelstoffwechsel sowie auch für die korrekte Funktion des Immunsystems. Die Verwendung von Hafer für die erfindungsgemäße Zahnpflegefuttermittelzusammensetzung bewirkt vorteilhaft eine höhere Aufnahme von Lysin durch ein Tier. Dies ist bspw. dann vorteilhaft, wenn die sonstigen, nicht von der Erfindung umfassten, Futtermittelbestandteile nur einen geringen Gehalt an Lysin aufweisen. Dies ist insbesondere bei pflanzlicher Ernährung der Fall, da Lysin in pflanzlichen Lebensmitteln in geringeren Konzentrationen vorkommt als in tierischen Produkten.

Das Haftmittel weist einen Zuckeranteil unter 5 Gew.-%, bevorzugt unter 3 Gew.-%, besonders bevorzugt unter 2 Gew.-%, ganz besonders bevorzugt unter 1,5 Gew.-%, weiter bevorzugt unter 1 Gew.-%, weiter besonders bevorzugt 0,5 Gew.-% oder weniger und weiter ganz besonders bevorzugt weniger als 0,2 Gew.-% auf. Die Angabe in Prozent bezieht sich hierbei auf das Gesamtvolumen des Haftmittels, bevorzugt aber auf das Gesamtgewicht des Haftmittels. So bedeutet ein Zuckeranteil von 1 Gew.-%, dass 1 g von 100 g Haftmittel einen Zucker umfasst. Dem Fachmann ist klar, dass der Zuckeranteil eines wasserhaltigen Haftmittels abhängig von dessen Wassergehalt ist. Ein Trocknen des Haftmittels bewirkt ein Verdunsten des enthaltenen Wassers, wodurch der Zuckeranteil steigt. Demnach bezieht sich der oben geforderte Zuckeranteil auf das Gewicht und/oder das Volumen des nicht getrockneten Haftmittels.

Nach der aktuell geltenden EU-Verordnung (EG 1924/2006) darf ein Lebensmittel oder eine Nahrungsmittelzusammensetzung als zuckerfrei deklariert werden, wenn diese einen Zuckergehalt von 0,5 % oder weniger beinhaltet. Im Sinne der Erfindung wird unter "zuckerfrei" demnach ein Zuckergehalt von 0,5 % oder weniger verstanden.

Ein Zucker im Sinne der Erfindung umfasst Disaccharide (z.B. Saccharose), Monosaccharide (z.B. Glucose, Fruktose), Oligosaccharide, insbesondere Trisaccharide (z.B. Maltotriose, Raffinose), Tetrasaccharide (z.B. Maltotetraose), Pentasaccharide, Hexasaccharide, Heptasaccharide aber auch Decasaccharide, oder Mischungen daraus. Besonders bevorzugt umfasst der Zucker Disaccharide, Monosaccharide oder Mischungen daraus. Da das erfindungsgemäße Haftmittel einen Zuckeranteil von weniger als 2 % aufweist, ergibt sich daraus vorteilhaft, dass dieser verträglicher für das Zahnmaterial ist. Dabei werden geringere Zuckeranteile (von bspw. unter 1 %) als weiter vorteilhaft für die Zahngesundheit angesehen.

In einer Ausführungsform umfasst das Haftmittel ein Klebereiweiß. Ein solches umfasst bspw. Gluten. Gluten ist ein aus verschiedenen Eiweißen zusammengesetztes Gemenge, das in bestimmten Getreidearten (z.B. Weizen, Roggen, Gerste, Hafer, Dinkel,) vorkommt. Die Stärkekörner des Mehlkörpers eines Getreidekorns werden von den Klebereiweißen Gliadin und Glutenin umgeben. Bei Kontakt mit Wasser verbinden sich beide Eiweiße zu Gluten und nehmen dabei ein Vielfaches ihres Eigengewichtes an Wasser auf (Wasserbindefähigkeit). Mit einer höheren Wasserbindefähigkeit kann das Haftmittel vorteilhaft bspw. leichter verstrichen werden.

In einer Ausführungsform umfasst der Klebstoff ein Klebeprotein, das im biologischen Ausgangsmaterial enthalten ist und in einem wässrigen Medium, insbesondere bevorzugt in einem salzhaltigen Medium, löslich und/oder dispergierbar ist. In einer besonders bevorzugten Ausführungsform ist das Klebprotein Avenalin, ein Globolin-Ähnliches Protein, welches ausschließlich in Hafer vorkommt. Dies hat den Vorteil, dass die Haftwirkung im abgebunden (getrockneten) Zustand verbessert ist, da Proteine als Haftklebstoffe fungieren können und so die Haftfähigkeit des Verbundes stärken können. Zudem besitzen sie einen Nährwert und stehen als essenzielle Stickstoffquelle zur Verfügung.

In einer weiteren Ausführungsform umfasst die Zahnpflegefuttermittelzusammensetzung eine vierte Komponente, die aus einem Additiv ausgewählt ist. Unter einem Additiv wird hierin ein Zusatzstoff verstanden, welcher kein Futtermittelbestandteil oder Haftmittel ist. Ein Additiv kann einen Duftstoff umfassen, wobei dieser bevorzugt dazu eingerichtet ist, die Zahnpflegefuttermittelzusammensetzung einem Tier für deren Verzehr vorteilhaft attraktiver zu machen. So kann ein Duftstoff bspw. nach frischen Kräutern, Gras, Fisch, Früchten, Obst oder Fleisch riechen. Der Fachmann kann dabei aus einer Vielzahl an unterschiedlichen Duftstoffen auswählen und wird diese auf die Spezies abstimmen, für welche die Zahnpflegefuttermittelzusammensetzung gedacht ist.

In einer bevorzugten Ausführungsform ist die vierte Komponente, die vorzugsweise aus einem Additiv, wie hierin definiert, ausgewählt ist oder daraus besteht, der zweiten Komponente, insbesondere dem Futtermitteladditiv, und/oder der dritten Komponente, insbesondere dem Haftmittel, zugesetzt. Dies ist insbesondere von Vorteil, wenn die erste Komponente, insbesondere der Grundkörper, als Formkörper ausgebildet ist, der als eine verholzte Hauptachse einer Baumpflanze ausgebildet ist oder besteht daraus, da so ohne großen Aufwand das Additiv über die zweite und/oder die dritte Komponente auf die Oberfläche der ersten Komponente aufgebracht werden kann.

Ein Additiv kann auch einen Zuckerersatzstoff (z.B. Stevia-Extrakt) umfassen, um die Zahnpflegefuttermittelzusammensetzung für das Tier geschmacklich attraktiver zu machen.

In einer besonders bevorzugten Ausführungsform enthält die gesamte Zusammensetzung einen Gesamtzuckergehalt der essbaren Bestandteile der Zahnpflegefuttermittelzusammensetzung, insbesondere des Grundkörpers, des Haftmittels und des Futtermitteladditivs, von unter 2 %.In einer weiteren Ausführungsform umfasst ein Additiv eine pharmazeutisch wirksame Substanz (auch: Medikament). Unter einer solchen wird zumindest eine chemische Verbindung verstanden, welche für eine therapeutische Maßnahme verabreicht wird. Kranken Tieren ein Medikament zu verabreichen, stellt den Halter oft vor Herausforderungen. Mit der Beimischung eines Additivs zu der Zahnpflegefuttermittelzusammensetzung kann vorteilhaft erreicht werden, dass ein Tier das Medikament auf einem einfachen Wege verabreicht bekommt.

Eine pharmazeutisch wirksame Substanz kann auch ein Beruhigungsmittel umfassen. So ist denkbar, dass die Zahnpflegefuttermittelzusammensetzung einem Pferd vor einem Transport des Pferdes dargeboten wird. Durch die Aufnahme des Beruhigungsmittels wird das Pferd ruhiger und kann somit vorteilhaft entspannter einer medizinischen Behandlung oder Schlachtung zugeführt werden. Dem Fachmann ist eine Vielzahl an Beruhigungsmitteln (z.B. Opioide, Benzodiazepine, Antidepressiva, Neuroleptika, Barbiturate, Thiazole, Betablocker, Phytopharmaka) bekannt, welche als Additiv Verwendung finden können.

Ein Additiv kann weiterhin eine Substanz umfassen, welche zusätzlich zur ersten Komponente ebenfalls dazu eingerichtet ist, einem Abrieb von Zahnmaterial förderlich zu sein. Beispielsweise könnte ein Additiv ein Pulver oder ein Granulat (z.B. Salzkörner, Sand, gemahlene Muschelschalen) umfassen. Weiterhin kann ein Additiv ein Silikat oder eine Silikatverbindung umfassen, um vorteilhaft den Zahnmaterialabrieb zu fördern.

Es sind zudem noch weitere Komponenten denkbar. So kann eine fünfte Komponente einen Geschmackstoff umfassen, wobei auch hier gilt, dass dieser vom Fachmann frei ausgewählt werden kann, je nachdem auf welche Spezies der Fachmann die Zahnpflegefuttermittelzusammensetzung abstimmt.

Eine vierte und/oder weitere Komponente (z.B. fünfte, sechste, siebte, etc.) kann, muss aber nicht, über ein Haftmittel mit zumindest einer der anderen Komponenten verbunden sein. Eine vierte und/oder weitere Komponente kann auch über ein Beschichtungsverfahren (z.B. Bestreichen, Besprühen, Eintauchen in ein Bad) mit zumindest einer der vorgenannten Komponenten verbunden werden.

Unter einer Verbindung wird hierin bevorzugt eine stoffschlüssige Verbindung verstanden. Dabei ist das Haftmittel so eingerichtet, dass dieses eine stoffschlüssige Verbindung zumindest einer Komponente (z.B. erste Komponente, insbesondere ein Grundkörper) mit einer weiteren Komponente (z.B. zweite Komponente, insbesondere ein Futtermitteladditiv) erlaubt. Unter einer stoffschlüssigen Verbindung wird hierin ein Kleben, Verkleben, Aneinanderkleben, Adhärieren verstanden. Bevor eine stoffschlüssige Verbindung etabliert werden kann, muss das Haftmittel mit zumindest einer der anderen Komponenten in Kontakt gebracht werden, d.h. es muss die Oberfläche zumindest einer Komponente kontaktieren und ist bevorzugt auf dieser verteilt.

In einer Ausführung der vorliegenden Erfindung wird ein Verfahren zur Herstellung einer Zahnpflegefuttermittelzusammensetzung beschrieben, welche folgenden Schritte umfasst. Die Bereitstellung der ersten Komponente, insbesondere einem Grundkörper, die Bereitstellung der zweiten Komponente, insbesondere einen Futtermitteladditiv, die Bereitstellung der dritten Komponente, insbesondere eines Haftmittels und das Inkontaktbringen der dritten Komponente mit der ersten und der zweiten Komponente, wobei durch die dritte Komponente eine stoffschlüssige Verbindung ausbildbar ist. Dabei kommt es durch das Inkontaktbringen der dritten Komponente mit der ersten und der zweiten Komponente im flüssigen oder viskosen Zustand. Anschließend kommt es bei dem Verfahren ferner zu einer anschließenden Aushärtephase, durch welche die stoffschlüssige Verbindung etabliert wird.

In einem Ausführungsbeispiel kann das Haftmittel bspw. im flüssigen Zustand über ein Bestreichen der ersten Komponente auf dieser aufgebracht werden, wobei die erste Komponente zumindest teilweise mit dem Haftmittel beschichtet wird. Auf die so aufgetragene Schicht kann die zweite Komponente aufgetragen werden. Im Anschluss erfolgt das Ausbilden einer stoffschlüssigen Verbindung durch bspw. Trocknen des Haftmittels. Da dieses wie weiter unten beschrieben, aus einem Getreide gewonnen wird, enthält es Stärke, welche im Wesentlichen zur stoffschlüssigen Verbindung beiträgt.

In einer alternativen Ausführungsform wird als erste und/oder zweite Komponente ein Granulat verwendet. Die Körnergröße des Granulats ist gleich oder kleiner als 10 mm, bevorzugt gleich oder kleiner als 8 mm, besonders bevorzugt gleich oder kleiner als 5 mm, ganz besonders bevorzugt gleich oder kleiner als 3 mm und weiter bevorzugt gleich oder kleiner als 2 mm. Die zweite Komponente kann mit der ersten und/oder einer dritten und/oder vierten und/oder weiteren Komponente stoffschlüssig verbunden werden, indem diese zusammen mit der dritten Komponente, die flüssig oder viskos vorliegen kann, zusammengepresst wird. Im Anschluss folgt eine Aushärtephase, um die stoffschlüssige Verbindung zu etablieren. Mit dem erfindungsgemäßen Haftmittel können so bspw. Futtermittelpellets hergestellt werden.

Eine Aushärtephase bezeichnet hierin einen Vorgang, der eine stoffschlüssige Verbindung des Haftmittels mit zumindest einer der anderen Komponenten ermöglicht. Eine durch ein hierin beschriebenes getreidebasiertes Haftmittel bewirkte stoffschlüssige Verbindung kommt im Wesentlichen dadurch zustande, indem der enthaltene Wasseranteil nach dem Auftragen/Beschichten des Haftmittels verdunstet. Eine Aushärtephase kann durch eine Temperatureinwirkung (z.B. Backen) und/oder durch den Feuchtigkeitsgehalt der umgebenden Luft gesteuert werden. Der Fachmann kann diese Parameter nach Bedarf mit entsprechenden Vorrichtungen (z.B. Backofen, Vakuumkammer) anpassen.

In einer besonders bevorzugten Ausführungsform ist die Zahnpflegefuttermittelzusammensetzung so ausgeführt, dass das Produkt kompakt, aber wasserdampfdurchlässig ist. Insbesondere die zweite Komponente, z.B. ein Mix aus unterschiedlichen Kräutern, Blüten, Sämereien, ist erfindungsgemäß porös und/oder weist ausreichend Lücken zwischen den festen wasserundruchlässigen Komponenten (z.B. Sämereien) auf, damit das Haftmittel, vorzugsweise Wasser, physikalisch abbinden kann.

Eine dritte Komponente und/oder eine vierte Komponente kann im nicht ausgehärteten Zustand, also vor der Aushärtephase, in flüssiger oder viskoser Form vorliegen. Dabei sind flüssig und viskos nicht klar voneinander abgrenzbar, sondern beschreiben vielmehr eine subjektiv vorgenommene Bewertung der Fließfähigkeit eines Stoffes oder einer Stoffzusammensetzung. Wasser wird beispielsweise als flüssig wahrgenommen, während Honig als viskos beurteilt wird.

In einer alternativen Ausführungsform wird das Haftmittel und die zweite Komponente (Nahrungsergänzungsmittel/ Futtermitteladditiv) und/oder weitere Komponenten als wirkungsmäßige Einheit gesehen. Vorzugsweise bilden das Futtermitteladditiv und das Haftmittel eine wirkungsmäßige Einheit, die eine Nahrungsergänzung mit positiven Eigenschaften für das Tier bereitstellt. Wird ein Biopolymer-basierte Haftmittel, bspw. ein Hafer-basiertes Haftmittel, bestehend im Wesentlichen aus der Protein-Stärke Matrix sowie weiteren wasserlöslichen und/oder dispergierbaren Bestandteilen, verwendet, haben die Bestandteile der Mischung einen positiven Effekt auf die Verträglichkeit der zweiten Komponente, vor allem für den als natürlich empfunden Geschmack als auch für eine gute Verdaulichkeit der Komponenten sowie gesundheitliche Aspekte wie die oben ausgeführte Cholesterin-Senkende Wirkung. Das Haftmittel ist in dieser bevorzugten Ausführungsform supplementiert somit die Wirkung der zweiten Komponente und hat einen wichtigen Einfluss auf die gewünschte Wirkung der Zahnpflegefuttermittelzusammensetzung.

In einer bevorzugten alternativen Ausführungsform wird das Haftmittel zusammen mit der und der zweiten Komponente (Nahrungsergänzungsmittel/Futtermitteladditiv) und/oder weiteren Komponenten aufgebracht und als einheitliche Flüssigkeit und/oder Suspension und/oder Aufschlämmung und/oder Paste auf den Grundkörper aufgebracht. Dadurch wird ein homogeneres Produkt erzeugt und eine variable und einstellbare Schichtdicke erreicht. Der Klebstoff wird dadurch Teil des Nahrungsergänzungsmittels und fördert so durch die erfindungsgemäßen Eigenschaften das Wohlbefinden des Tieres.

Als ein Maß für die Fließfähigkeit eines Stoffes oder Stoffgemisches kann die dynamische Viskosität gewählt werden. Die dynamische Viskosität beschreibt die Zähigkeit von Flüssigkeiten. Sie wird definiert durch den Reibungswiderstand, den eine Flüssigkeit einer Verformung durch eine Druck- oder Schubspannung entgegensetzt. Die dynamische Viskosität η (griechisch Buchstabe eta) wird im Allgemeinen in Millipascalsekunden (mPas) angegeben und mittels rheologischer Messungen bestimmt. Da die Temperatur maßgeblich die Viskosität eines Stoffes oder Stoffgemisches beeinflusst, muss diese zusätzlich angegeben werden.

Im Sinne der Erfindung wird unter einer flüssigen Komponente (z.B. Haftmittel, Additiv) ein solches verstanden, welches einer Temperatur von 0 °C bis 60 °C eine dynamische Viskosität (η) zwischen 1 und 100.000 mPas, bevorzugt zwischen 1 und 50.000 mPas, besonders bevorzugt zwischen 1 und 20.000 mPas, ganz besonders bevorzugt zwischen 1 und 10.000 mPas und weiter bevorzugt zwischen 1 und 5.000 mPas aufweist.

Beispielsweise weist Wasser bei 20 °C eine dynamische Viskosität (η) von 1,0 mPas auf. Honig weist bei 20 °C eine dynamische Viskosität von 10.000 mPas auf. Butter weist bei 40 °C eine dynamische Viskosität von 30.000 mPas auf. Teer weist bei 20 °C eine dynamische Viskosität von 100.000 mPas auf. Mit einer geringeren dynamischen Viskosität geht vorteilhaft bspw. eine einfachere Beschichtung einer Komponente mit der dritten Komponente einher.

In einer bevorzugten Ausgestaltung wird zumindest eine der Komponenten, bspw. die erste, zweite, vierte Komponente, gereinigt und/oder getrocknet. Bevorzugt findet eine Reinigung einer Komponente ohne die Mitwirkung chemischer Zusätze (z.B. Reinigungsmittel) statt. So wird insbesondere die Reinigung durch Abbürsten und/oder Abschleifen und/oder Abspülen (z.B. mit Wasser) als vorteilhaft angesehen, da somit keine Reinigungsmittel von einem Tier aufgenommen werden. Dem Fachmann sind weitere Reinigungsverfahren (z.B. mechanische Reinigungsverfahren wie z.B. Abschleifen) bekannt, welche vorteilhaft auf die Verwendung eines Reinigungsmittels verzichten.

In einer weiteren Ausführungsform wird insbesondere die erste Komponente (z.B. ein Stück eines Baumstammes) getrocknet, wobei auch weitere Komponenten getrocknet werden können. Nach der Trocknung liegt der Restfeuchtegehalt (auch: Holzfeuchtegehalt) des Holzes bei unter 40 %, bevorzugt unter 30 %, besonders bevorzugt bei gleich oder unter 20 %, ganz besonders bevorzugt bei unter 15 %, weiter bevorzugt bei unter 10 %, weiter besonders bevorzugt bei unter 5 %, jeweils vorzugsweise bezogen auf das Gesamtgewicht der ersten Komponente.

Als Holzfeuchte oder Holzfeuchtigkeit wird das Verhältnis der im Holz enthaltenen Wassermasse zur Trockenmasse des Holzes in Prozent bezeichnet. Die Holzfeuchte ist nicht zu verwechseln mit dem Wassergehalt des Holzes, welche das Verhältnis von der im Holz enthaltenen Wassermasse zur Gesamtmasse des (feuchten) Holzes in Prozent wiedergibt.

Dem Fachmann sind Verfahren bekannt, die zur Trocknung von Holz verwendet werden können. Beispielsweise kann ein Holz über einen Wärmeeintrag (z.B. 100 °C) über einen bestimmten Zeitraum (z.B. 8 Stunden) getrocknet werden. Alternativ kann auch eine Vakuumtrocknung Anwendung finden. Vorteilhaft an einer Vakuumtrocknung ist ein geringerer Energieverbrauch als bei einer Trocknung mittels Wärmeeintrag.

Mit einer Reinigung und/oder Trocknung zumindest einer Komponente wird vorteilhaft erreicht, dass bspw. ein Holz frei von Pilzbefall und/oder Schimmel ist. Somit weist die Zahnpflegefuttermittelzusammensetzung ein geringeres Krankheitserregungspotential auf.

Abschließend sei angemerkt, dass sämtlichen Merkmalen, die in den Anmeldungsunterlagen und insbesondere in den abhängigen Ansprüchen genannt sind, trotz des vorgenommenen formalen Rückbezugs auf einen oder mehrere bestimmte Ansprüche, auch einzeln oder in beliebiger Kombination eigenständiger Schutz zukommen soll.

Weitere Vorteile, Merkmale und Anwendungsmöglichkeiten der vorliegenden Erfindung ergeben sich auch aus der nachfolgenden Beschreibung von Ausführungsbeispielen. Dabei bilden alle beschriebenen Merkmale für sich oder in beliebiger Kombination den Gegenstand der vorliegenden Erfindung, auch unabhängig von ihrer Zusammenfassung in den Ansprüchen oder deren Rückbezügen.

Zudem soll darauf hingewiesen werden, dass der Fachmann zweifelsohne erkennt, dass sich die einzelnen Merkmale, die in den vorstehenden konkreten Ausführungsformen beschrieben sind, auf angemessene Weise miteinander kombinieren lassen, soweit kein Widerspruch vorliegt, wobei zum Vermeiden unnötiger Wiederholung auf eine separate Beschreibung verschiedener möglicher Kombinationen verzichtet wird.

Die Erfindung umfasst ferner die Verwendung der dritten Komponente, insbesondere des Haftmittels, zur stoffschlüssigen Verbindung von zumindest einer der oben genannten Komponenten.

Darüber hinaus umfasst die Erfindung ferner die Verwendung der dritten Komponente, insbesondere des Haftmittels (wie hierin definiert), als Haftmittel in einer Futtermittelzusammensetzung, insbesondere in einer Zahnpflegefuttermittelzusammensetzung.

### AUSFÜHRUNGSBEISPIELE

Nachfolgend wird die vorliegende Erfindung anhand von Ausführungsbeispielen näher erläutert.

### Dabei zeigt

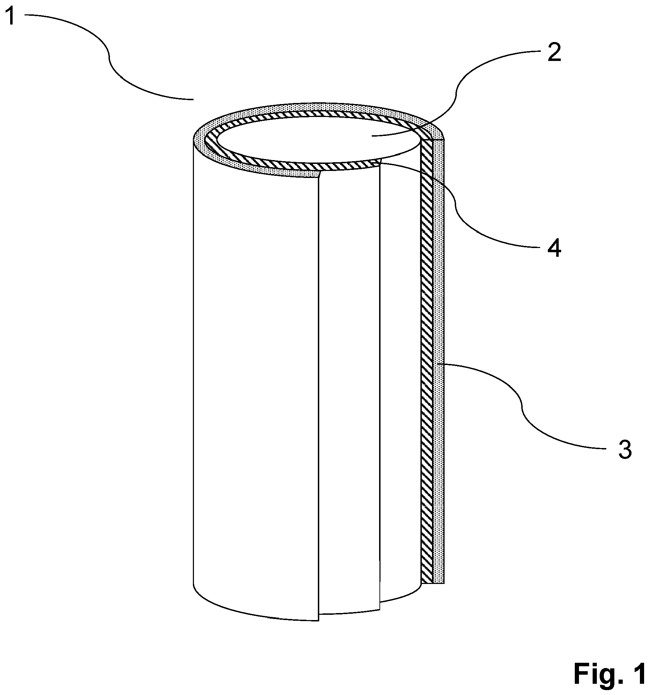

- Fig. 1:: eine schematische Schnittzeichnung durch die Schichten einer Zahnpflegefuttermittelzusammensetzung (1), nämlich ein Futtermitteladditiv (3), ein darunter angeordnetes Haftmittel (4), welches auf der Mantelfläche eines formgebenden Grundkörpers (2) angeordnet ist.

In einer Ausführungsform umfasst die Zahnpflegefuttermittelzusammensetzung als erste Komponente ein Holzteil eines Stammes aus sibirischer Birke, wobei das Teil zylindrisch ist, einen Durchmesser von 12 cm und eine Länge von 30 cm aufweist und wobei die Rinde des Baumes erhalten ist. Die Abmessung und Form des Holzteils kann auch hiervon abweichen. Bspw. kann das Holzteil zylindrisch sein und einen Durchmesser von 5 cm und eine Länge von 15 cm aufweisen. Alternativ sind auch scheibenförmige Holzteile mit einer Dicke von 2 cm denkbar.

Da die Rinde eines Baumes das Phloem enthält, sind in der Rinde Spurenelemente vorhanden, die von dem Pferd aufgenommen werden können, während es an dem Holzteil knabbert. Das Holzteil wurde zuvor für 5 Stunden einer Hitzebehandlung von 120 °C unterzogen, um dem Holz Feuchtigkeit zu entziehen.

In dieser Ausführungsform wird die dritte Komponente (das Haftmittel) erzeugt, indem 20 g Haferflocken mit 200 ml Wasser vermengt werden. Die Vermengung erfolgt innerhalb eines Durchschlagsiebs, welcher in einem Kochtopf angeordnet ist. Dabei ist darauf zu achten, dass die Haferflocken, welche sich innerhalb des Siebs befinden, zumindest teilweise, bevorzugt zumindest zu einem Drittel, in das Wasser eingetaucht sind. Die Porengröße des Durchschlagsiebes ist so gewählt, dass die Haferflocken bevorzugt nicht hindurchpassen.

Hiernach wird das Wasser erhitzt, bis es zu sieden beginnt. In diesem Beispiel erfolgt das Sieden nach etwa 10 Minuten bei einer Temperatur von 94,8 °C. Während des Erhitzens wird die Haferflockenmasse ununterbrochen in dem Durchschlagsieb (z.B. mit einem Kochlöffel) verrührt. Gleichzeitig wird die Flüssigkeit im Topf (außerhalb des Durchschlagsiebes) mit (z.B. einem Schneebesen) umgerührt, damit diese nicht anbrennt. Bei dieser Flüssigkeit handelt es sich um eine Vorstufe des sich ergebenden Haftmittels (auch: Haftmittelvorstufe).

Der Vorgang wird so lange durchgeführt (in diesem Beispiel etwa 20 Minuten), bis das Wasser im Topf zu einem Teil, zumindest jedoch zu 30 %, verdampft ist. Die Haftmittelvorstufe wird so lange gekocht, bis diese zwar dickflüssig, aber immer noch streichfähig ist und in etwa der Konsistenz von Joghurt entspricht. (Die dynamische Viskosität von Joghurt entspricht bei 40°C etwa 150 mPas.)

Die Haftmittelvorstufe wird im Anschluss durch ein Sieb mit einer Porengröße von 1 mm oder durch eine Mullwindel abgeseiht, um Klümpchen im Haftmittel zu vermeiden. Das Haftmittel wird von Zeit zu Zeit umgerührt, damit es nicht vorzeitig (vor dem Auftragen) fest wird.

Das sich ergebende Haftmittel kann nun auf das Holzteil aufgetragen werden. Das Haftmittel wird bevorzugt zum Bestreichen verwendet, bevor es abgekühlt ist. Die auf das Holzstück aufgetragene Schicht des Haftmittels ist etwa 1 mm dick.

Im Anschluss daran kann die zweite Komponente (z.B. ein Mix aus unterschiedlichen Kräutern, Blüten, Sämereien) auf das noch nicht ausgehärtete Haftmittel aufgetragen werden (bspw. über Bestreuen), worauf eine Aushärtephase für 6 Stunden bei Raumtemperatur durchgeführt wird, während das Haftmittel fest und die stoffschlüssige Verbindung etabliert wird.

Die beschriebenen Ausführungsformen der Erfindung sind jeweils beispielhaft und nicht beschränkend zu verstehen. Die Erfindung kann auch auf hiervon abweichende Weise ausgeführt werden. Beispielsweise können die verwendeten Volumina an Wasser oder die Menge der verwendeten Haferflocken variiert werden. Weiterhin kann auch die Temperatur und Dauer des Siedevorgangs, sowie Porengröße der Siebe variiert werden.

Die im oben beschriebenen Ausführungsbeispiel genannten Volumina, Gewichte, Temperaturen, dynamische Viskositäten, Porengrößen und Zeiten können in alternativen Ausführungsformen abweichen. Die Abweichung jeder dieser Größen kann sich dabei in einem Bereich von 200 %, bevorzugt 100 %, besonders bevorzugt 50 % und ganz besonders bevorzugt um 25 % um eine der oben angegebenen Größen bewegen.

Es ist ebenso denkbar, dass das oben beschriebene Verfahren nicht manuell, sondern in einem großtechnischen Maßstab durchgeführt wird, wobei dem Fachmann geeignete Verfahrensanordnungen bekannt sind, um das oben beschriebene Verfahren mittels einer technischen Vorrichtung automatisiert auszuführen. So kann der Fachmann sämtliche physikalischen Parameter verändern, um aus einem Biopolymer-basierte Haftmittel, bspw. aus Getreide, vorzugsweise aus Mais, Reis, Erbsen, Hirse, Amarant und/oder Hafer, besonders bevorzugt aus Hirse, Amarant und/oder Hafer, insbesondere aus Hafer, das erfindungsgemäße Haftmittel herzustellen.

Die vorstehenden Ausführungen sind nur einige bevorzugte und realisierbare Ausführungsformen der vorliegenden Erfindung. Daher sind alle gleichwertigen strukturellen Änderungen, die durch Anwendung der Beschreibung der Erfindung vorgenommen werden, in den Umfang der Patentanmeldung einzubeziehen.

Der Fachmann erkennt, dass Variationen und Modifikationen vorgenommen werden können, ohne dass der wahre Umfang der Erfindung, wie er durch die Ansprüche und Beschreibung definiert ist, verlassen wird. Es ist daher beabsichtigt, im Rahmen der Erfindung alle Variationen und Modifikationen, die in den Anwendungsbereich der beigefügten Ansprüche und deren Äquivalente fallen, einzuschließen.

### BEZUGSZEICHENLISTE

- 1: Zahnpflegefuttermittelzusammensetzung
- 2: erste Komponente, bspw. ein formgebender Grundkörper
- 3: zweite Komponente, bspw. ein Futtermitteladditiv
- 4: dritte Komponente, bspw. ein Haftmittel

## Patentansprüche

1. **Zahnpflegefuttermittelzusammensetzung** (1) zur Zahnpflege und Nahrungsergänzung eines Tieres, umfassend zumindest folgende drei Komponenten:
- einen formgebenden Grundkörper (2), der zumindest ein Material umfasst, welches zum Abrieb des Zahnmaterials des Tieres geeignet ist,
- ein Futtermitteladditiv (3), das zumindest einen Futtermittelbestandteil umfasst,
- ein Haftmittel (4), das ein verzehrbares Haftmittel umfasst, oder daraus besteht, welches zur stoffschlüssigen Verbindung aller Komponenten eingerichtet ist,
**dadurch gekennzeichnet, dass**
das Haftmittel (4) ein Haftmittel auf Biopolymerbasis ist, das aus einer Pflanze extrahiert wird und einen Zuckergehalt von weniger als 2 % aufweist.

2. Zusammensetzung gemäß Anspruch 1, wobei das Haftmittel (4) zumindest auf einem Teil der Oberfläche des Grundkörpers (2), insbesondere teilweise als Schicht auf einem Teil der Oberfläche des Grundkörpers (2), angeordnet ist.

3. Zusammensetzung gemäß einem der Ansprüche 1 oder 2, wobei das Biopolymer-basierte Haftmittel (4) Stärke-Partikel aufweist, wobei die mittlere Partikelgröße der Stärke-Partikel in dem Extrakt der Pflanze, insbesondere vor dessen Verarbeitung zur Zahnpflegefuttermittelzusammensetzung, besonders bevorzugt vor dessen Verarbeitung wie hierin beschrieben, zwischen 1-15 µm, bevorzugt bei 1-12 µm, liegt.

4. Zusammensetzung gemäß einem der Ansprüche 1 bis 3, wobei das Biopolymer-basierte Haftmittel (4) Stärke-Partikel und/oder Mischungen davon aufweist, bei denen der Gelatinierungsbereich breiter ist als 10 K.

5. Zusammensetzung gemäß einem der Ansprüche 1 bis 4, wobei die dritte Komponente, insbesondere das Haftmittel (4), einen Lipidgehalt von etwa 1,0 Gew.-% (±0,05) und mehr aufweist und/oder bevorzugt ein Klebereiweiß enthält.

6. Zusammensetzung gemäß einem der Ansprüche 1 bis 5, wobei das Biopolymer-basierte Haftmittel (4) Stärke-Partikel und/oder Mischungen davon aufweist, bei denen zumindest eine Peaktemperatur oberhalb von 60 °C, besonders bevorzugt oberhalb von 65 °C, ganz besonders bevorzugt oberhalb von 70 °C, insbesondere oberhalb von 74 °C liegt.

7. Zusammensetzung gemäß einem der Ansprüche 1 bis 6, wobei der Grundkörper (2) mit dem Haftmittel (4) teilweise oder vollständig umgeben ist und auf der Außenseite des Haftmittels (4) das Futtermitteladditiv (3) adhäsiert ist.

8. Zusammensetzung gemäß einem der Ansprüche 1 bis 7, wobei die Zusammensetzung eine vierte Komponente umfasst, die aus einem Additiv, wie zum Beispiel eine pharmazeutisch wirksame Substanz, ausgewählt ist.

9. **Verfahren** zur Herstellung einer Zahnpflegefuttermittelzusammensetzung (1) gemäß einem der Ansprüche 1 bis 8, umfassend die folgenden Schritte:
a) Bereitstellen eines formgebenden Grundkörpers (2), der zumindest ein Material umfasst, welches zum Abrieb des Zahnmaterials des Tieres geeignet ist,
b) Bereitstellen zumindest eines Futtermitteladditives (3), das zumindest einen Futtermittelbestandteil umfasst
c) Bereitstellen eines Haftmittels (4) nach Anspruch 1,
d) Inkontaktbringen des Grundkörpers (2) mit dem Haftmittel (4) und dem Futtermitteladditiv (3), wobei durch das Haftmittel (4) eine stoffschlüssige Verbindung ausgebildet wird,
**dadurch gekennzeichnet, dass**
das Haftmittel (4) beim Inkontaktbringen mit dem Grundkörper (2) und/oder dem Futtermitteladditiv (3) im flüssigen oder viskosen Zustand ist,
wobei das Verfahren ferner eine sich anschließende Aushärtephase umfasst, durch welche die stoffschlüssige Verbindung etabliert wird.

10. Verfahren gemäß Anspruch 9, wobei zusätzlich eine vierte Komponente gemäß Anspruch 8 bereitgestellt wird, wobei die vierte Komponente mit zumindest einer der oben genannten Komponenten stoffschlüssig verbunden wird.

11. Verfahren gemäß Anspruch 9 oder 10, wobei der Grundkörper (2) und/oder das Futtermitteladditiv (3) und/oder die vierte Komponente vor dem Bereitstellen für das Verfahren nach Anspruch 9 oder 10 gereinigt und/oder getrocknet wird, wobei besonders bevorzugt der Restfeuchtegehalt des Grundkörpers (2) und/oder der Futtermitteladditivs (3) und/oder der vierten Komponente bei unter 30 % liegt.

12. Verfahren gemäß einem Anspruch 9 bis 11, in der das Biopolymer, aus welchem das Haftmittel (4) besteht, bevorzugt oberhalb von 80 °C, besonders bevorzugt im Bereich von 90 °C bis 110 °C, am meisten bevorzugt zwischen 95 °C bis 105 °C mit einem polaren Lösungsmittel, vorzugsweise Wasser, aus einer pflanzlichen Quelle, besonders bevorzugt aus Hafer, extrahiert wird.

13. Verfahren gemäß einem der Ansprüche 9 bis 12, wobei das Futtermitteladditiv (3) und/oder die vierte Komponente mit dem Haftmittel (4) im flüssigen oder viskosen Zustand vermengt werden und als Einheit auf den Grundkörper (3) aufgetragen werden.

14. **Zahnpflegefuttermittelzusammensetzung** hergestellt nach einem Verfahren gemäß einem der Ansprüche 9 bis 13.

15. **Verwendung** des Haftmittels nach einem der Ansprüche 1 bis 8 als Haftmittel in einer Futtermittelzusammensetzung.
